# EUROPÄISCHE PATENTANMELDUNG

(11) **EP 1 967 066 A2**
(43) Veröffentlichungstag der Anmeldung: **10.09.2008**
(21) Anmeldenummer: 08003877.1
(22) Anmeldetag: 03.03.2008
(51) Int. Cl.: A01N 31/02, A01N 25/30, A01P 1/00

(54) **Alkoholhaltiges Mittel und Verfahren zur mikrobiologischen Schnell-Desinfektion unbelebter Oberflächen im Sekundenbereich**

(30) Priorität: 07.03.2007 DE 102007010939
(71) Anmelder: Merz Pharma GmbH & Co. KGaA, 60318 Frankfurt Main (DE)
(72) Erfinder: Knebl, Robert, Dr., 71549 Auenwald (DE); Lindner, Dirk, 63303 Dreieich-Offenthal (DE)
(74) Vertreter: Müller, Claudia

(57) **Zusammenfassung**

Die vorliegende Erfindung betrifft ein Mittel zur manuellen Desinfektion von unbelebten Oberflächen, wie solchen in ärztlichen, zahnärztlichen Praxen, welches eine mikrobiologische Wirksamkeit innerhalb eines Zeitraums von weniger als 30 Sekunden aufweist und alkoholhaltig ist.
Überraschenderweise wurde gefunden, dass alkoholhaltige Desinfektionsmittel auf unbelebten Oberflächen wirksam eingesetzt werden können, wenn die Einwirkzeit hierfür weniger als 30 Sekunden beträgt. Die alkoholhaltigen Mittel können weitere Zusätze aufweisen, sie können vorzugsweise frei sein von Polyalkylenglykolen und / oder sauren Phosphorsäurederivaten und / oder Mono-Alkylglycerinethem.

## Beschreibung

### Gegenstand der Anmeldung

Die vorliegende Erfindung betrifft ein Mittel und Verfahren zur umfassenden insbesondere manuellen Desinfektion von unbelebten Oberflächen, wie solchen in ärztlichen, zahnärztlichen Praxen, von entsprechendem Mobilar und

Ausstattungsgegenständen, welches eine mikrobiologische Wirksamkeit innerhalb eines Zeitraums von weniger als 30 Sekunden aufweist und alkoholhaltig ist.

Überraschenderweise wurde gefunden, dass alkoholhaltige Desinfektionsmittel auf unbelebten Oberflächen wirksam eingesetzt werden können, wenn die Einwirkzeit hierfür weniger als 30 Sekunden beträgt. Dies steht im Gegensatz zu bisher angewendeten Mitteln, insbesondere bei der Hand- und Hautdesinfektion, für welche Einwirkzeiten von mindestens 30 Sekunden und mehr angegeben werden, obgleich auf lebenden Zellen wie Haut Desinfektionsmittel normalerweise besser wirken können, weil Alkohol und andere Stoffe als Penetrationsförderer vorhanden sind. Auf unbelebten, insbesondere glatten Oberflächen ist eine derartige Penetration nicht möglich. Dennoch können unerwarteter Weise Oberflächen gemäß vorliegender Erfindung in kürzester Zeit desinfiziert werden. Dabei sind mikrobizide Wirkstoffe wie quaternäre Ammoniumverbindungen und

Analoga wie Chlorhexidin, Benzalkoniumchlorid nicht zwingend erforderlich. Das alkoholhaltige Mittel kann bevorzugt auch frei sein von Polyalkylenglykolen, auch müssen saure Phosphorsäurederivate und / oder Glycerinmonoalkylether nicht enthalten sein.

### Stand der Technik

Mittel zur Desinfektion von unbelebten Oberflächen sollen praxisorientiert sein. Dies bedeutet, dass sie einfach in der Anwendung und schnell bzgl. ihrer Wirksamkeit sind. Als Applikationsformen kommen dabei vor allem manuelles Auftragen, Sprühen, Reiben mit vorgetränkten Tüchern zum Einsatz. Dabei treten bzgl. der optimalen Desinfektionswirkung oftmals Schwierigkeiten hinsichtlich der Gleichverteilung des Wirkstoffs auf. Für bekannte Alkohol- oder Alkohol und Biozid- haltige Mittel werden bei Testverfahren gemäß DGHM- Richtlinien meistens Einwirkzeiten von 1 bis 2 Minuten angegeben, ggf. 30 Sekunden in Anwesenheit von Glutaraldehyd, damit das gesamte Keimspektrum wie Pilze, Bakterien, Viren, insbesondere behüllte, abgedeckt werden kann. Allerdings werden im medizinischen Betrieb oftmals kürzere Einwirkzeiten erforderlich, z. B. auf Operationstischen, Zahnarztstühlen, anderen ärztlichen Behandlungsstühlen etc.

In der EP A 1 685 854 A1 wird ein alkohol- und insbesondere (Poly)Alkylenglykolverbindungen enthaltendes Mittel beschrieben, welches eine Einwirkzeit von etwa 1 Minute gegen Viren wie Polio, Vaccina bei der Handdesinfektion erfordert. Es sollen auch saure Phosphorsäurederivate /- verbindungen enthalten sein. Insbesondere wird auf die notwendige Anwesenheit von Alkylenglykol hingewiesen. Alkylenglykole wirken in Alkoholhaltigen Mitteln als Verdicker, ggf. auch als Penetrationsbeschleuniger oder Rückfetter.

In der DE 10 2005 002 645 A1 werden alkoholische Desinfektionsmittel offenbart, enthaltend mindestens einen Monoalkylglycderinether, nämlich 1- oder 2-(C₁-C₂₄-Alkyl)glycerinether (welcher ebenfalls als Rückfetter wirken kann) und ein oder mehrere Bispyridiniumalkane als bioziden Wirkstoff. Das Mittel soll insbesondere bei der Haut- und Händedesinfektion innerhalb von 30 Sekunden bis 1 Minute eingesetzt werden, wobei insbesondere die Kombination der beiden genannten Komponenten ausschlaggebend sein soll.

### Aufgabe vorliegender Erfindung

Aufgabe vorliegender Erfindung ist es daher, ein Mittel bereitzustellen, welches sicher und praxisorientiert in der Anwendung ist und darüber hinaus einen kürzest möglichen Wirksamkeitszeitraum aufweist.

### Lösung der Aufgabe

Diese Aufgabe wird erfindungsgemäß dadurch gelöst, dass man ein alkoholhaltiges Mittel für unbelebte Oberflächen bereitstellt, welches eine Einwirkzeit von weniger als 30 Sekunden, insbesondere zwischen 10 und 25 Sekunden aufweist.

Bevorzugt weist es ein oder mehrere amphotere Tenside oder NH₃ (Ammoniak, d. h. wässrige Lösungen hiervon wie z.B. 25 %ig) oder Mischungen hiervon auf.

Es ist innerhalb eines Zeitraums von weniger als 30 Sekunden auf unbelebten Flächen (mikrobiologisch) wirksam z. B. gegen Bakterien, Pilze, sowie behüllte und unbehüllte Viren, wie insbesondere grampositive oder gramnegative Bakterien wie z.B. Staphylococcus aureus, Pseudomonas aeruginosa und

Enterococcus hirae, (Keimreduktion bevorzugt log RF ≥ 5), oder auch gegen Pilze, insbesondere Hefen wie Candida albicans, sowie behüllte / unbehüllte Viren wie z. B. HIV, HBV, HCV, Rota- oder Vaccinaviren oder auch Mykobakterien (Keimreduktion bevorzugt log RF ≥ 4). Polyalkylenglykole sind nicht erforderlich und können vorzugsweise nicht enthalten sein. Auch sind saure Phosphorsäureverbindungen, insbesondere Phosphorsäurederivate und / oder Monoalkyl-Glycerinether (1- oder 2-(C₁-C₂₄-Alkyl)glycerinether) erfindungsgemäß nicht zwingend erforderlich und daher bevorzugt nicht enthalten.

### Nähere Erläuterung der Erfindung

Bevorzugt weist das Mittel gemäß vorliegender Erfindung einen oder mehrere aliphatische einwertige C₂-C₄-, insbesondere C₂-C₃- Alkohole, vor allem ausgewählt aus Ethanol, n- Propanol, Isopropanol oder Mischungen hiervon auf. Als Mischungen werden bevorzugt Ethanol / n- Propanol und / oder Isopropanol in einem Verhältnis von 10:1 bis 1:5, insbesondere bis 1:1. Es kann auch vorteilhaft sein, wenn die Menge an Ethanol höher als jene an n- Propanol und / oder Iso- Propanol ist, wie z.B. 10:1 bis 1,5:1, vor allem bis 2:1.

Die Gesamtmenge an Alkohol kann variieren und beträgt bevorzugt 35-75 Gew. %, insbesondere 38 bis 65 Gew. %, vor allem 42 bis 58 Gew.%.

Das Mittel weist ferner Wasser auf.

In einer weiteren Ausführungsform können 0,01 bis 15 Gew. %, bevorzugt 0,01 bis 10 Gew.% , insbesondere 0,01 -5 Gew.% oder auch 0,1 bis 5 Gew. %, eines oder mehrerer Zusätze, ausgewählt aus NH₃ (Ammoniak, d. h. wässrige Lösungen hiervon wie z.B. 25 %ig), zum Beispiel 0,01-0,1 %, einem oder mehreren amphoteren Tensiden, einem oder mehreren nicht ionischen Tensiden, einem oder mehreren pH- Wert Regulatoren, einem oder mehreren bioziden Wirkstoffen, Parfümstoffen, Farbstoffen, sowie Wasser (als Rest) vorhanden sein.

Besonderes bevorzugte Zusätze sind ausgewählt aus NH₃ (Ammoniak, d. h. wässrige Lösungen hiervon wie z.B. 25 %ig), amphoteren Tensiden oder auch Mischungen hiervon. Insbesondere bevorzugt sind als Zusatz ein oder mehrere amphotere Tenside.

Bevorzugte amphotere Tenside sind Alkylamidopropylbetaine, wie z. B. Cocoamidopropylbetain, N-Alkyl Aminopropyl Glycine, mit C₈-C₁₈, bevorzugt C₈ - C₁₂- Alkylgruppen, Sulfobetaine oder Imidazoliniumverbindungen, oder Mischungen hiervon.

Eine weitere Ausführungsform der Erfindung umfasst Mittel, enthaltend 45-60 % Alkohol, insbesondere Ethanol, Wasser sowie ein oder mehrere amphotere Tenside, insbesondere N-Alkylaminopropylglycin (mit Alkyl wie oben beschrieben), z. B. in einer Menge von 0,01 bis 2 Gew.%.

In einer weiteren bevorzugten Ausführungsform können als Zusatz auch ein oder mehrere biozide Wirkstoffe, in einer Menge wie nachfolgend genannt, bevorzugt quaternäre Ammoniumverbindungen oder Biguanidine wie nachfolgend genannt, oder Mischungen hiervon, eingesetzt werden.

In einer weiteren Ausgestaltung der Erfindung ist auch eine Kombination aus amphoterem Tensid und biozidem Wirkstoff möglich.

Weiterhin können gewünschtenfalls in diesen besonderen Ausgestaltungen vorliegender Erfindung Parfüm- und /oder Farbstoffe und / oder pH- Wert-Regulatoren hinzugefügt werden.

Eine weitere geeignete Ausführungsform sind Mittel, die 38 bis 65 Gew. % Alkohol, 0,01 bis 5 Gew. Zusätze sowie Wasser aufweisen.

Besonders bevorzugt sind Mittel, enthaltend Wasser, Ethanol / und 1 oder n-Propanol und / oder Isopropanol, sowie ein oder mehrere amphotere Tenside, vor allem ausgewählt aus den oben genannten bevorzugten, insbesondere Alkylamidopropylbetainen wie beschrieben.

Das oder die Tenside können sofern vorhanden, bevorzugt in einer Menge von 0,01- 4,0 %, insbesondere 0,01 bis 1 % oder auch 0,1 bis 1 Gew. % enthalten sein. Anionische Tenside, wie insbesondere Alkylsulfate, sind bevorzugt nicht enthalten.

Geeignete pH- Wert- Regulatoren können bevorzugt ausgewählt werden aus Säuren, Basen, z. B. organischen oder anorganischen Säuren, Basen, z. B. Milchsäure, Citronensäure, Apfelsäure, Essigsäure, Salzsäure, Amidosulfonsäure, Aminosäurederivaten oder Mischungen hiervon und in Mengen von z. B. 0,01 - 0,5% vorliegen.

Als Parfüm / Farbstoffe können hierfür gattungsgemäße Komponenten in üblichen dem Fachmann bekannten Mengen eingesetzt werden wie z. B. je z. B. 0,1 bis 0,5%, insbesondere ätherische Öle wie Orangenöl; oder andere hierfür bekannte Stoffe verwendet werden.

Bevorzugte biozide Wirkstoffe können ggf. in geringen Mengen wie 0,01 - 4%, insbesondere 0,01 bis 1,0 Gew. % oder auch 0,1 bis 1 Gew. %, je nach gewünschtem Ziel zugesetzt werden. Hierzu eignen sich kationische biozide Wirkstoffe, insbesondere langkettige quaternäre Ammonium- Verbindungen, und / oder Guanidine, wie Di-C₈ bis C₂₂-n-Alkyldimethylammonium - Verbindungen, Mono-n-C₈-C₁₈-Alkyl-dimethylbenzylammonium-Verbindungen, Benzalkoniumchlorid, / -Bromid / -Propionat oder -Acetat, Polyhexamethylenbiguanid, Octinidinhydrochlorid, Polyhexanid, Mecitroniumethylsulfat, Chlorhexidinsalze, Alkylpropylendiamin-1,5-bis-guanidinium-acectat; ein oder mehrere Amine, ausgewählt aus primären, sekundären und / oder tertiären Aminen, wie z. B. Bis-(3-aminopropyl)-laurylamin und / oder Laurylpropylendiamin oder heterozyclische Komponente/n wie Piperazin oder Mono- C₁-C₆- Aikyi - substituiertes Piperazin oder Mischungen derartiger Wirkstoffe. Bevorzugt kann bei Anwesenheit von quaternärem Wirkstoff und Amin das Verhältnis von Amin/en zu kationischem / n biozidem / n Wirkstoff/en gleich oder kleiner 1 (die Menge an Amin / en gleich oder geringer ist als die Menge an biozidem / n Wirkstoff/en) sein. Aldehyde werden bevorzugt nicht eingesetzt.

Bevorzugte biozide Wirkstoffe sind quaternäre Ammonium- Verbindungen, und / oder Biguanidine, wie Di- C₈ bis C₂₂ - n- Alkyldimethylammonium- Verbindungen,

Mono-n-C₈-C₁₈-Alkyl-dimethylbenzylammonium-Verbindungen, Polyhexamethylenbiguanid, Polyhexanid, oder Mischungen hiervon.

Als nichtionische Tenside eignen sich vor allem Aminoxide. Auch sind AlkylC₈-C₁₆ - Fettalkoholalkoxylate mit Alkoxylierungsgraden von 8 bis 20, insbesondere EO Graden von mehr als 14, wie Alkylfettalkoholethoxylate/ -propoxylate wie z. B.

Fettalkohol (C12)-ethoxylat (14EO), oder Mischungen hiervon mit Aminoxiden möglich.

In einer weiteren Ausführungsform umfasst das Mittel gemäß vorliegender Erfindung 35-70 Gew.%, vor allem 40-60 Gew. %, eines oder mehrerer Alkohole, vor allem ausgewählt aus Ethanol, n- Propanol, 0,01 bis 10 Gew. % , insbesondere 0,01 bis 5 Gew. %, eines oder mehrerer Zusätze, ausgewählt aus NH₃ (Ammoniak, d. h. wässrige Lösungen hiervon wie z.B. 25 %ig), einem oder mehreren amphoteren Tensiden, einem oder mehreren pH- Wert Regulatoren, sowie als Rest Wasser. Besonders bevorzugt sind als Zusatz ein oder mehrere amphotere Tenside.

Die Mittel der oben beschriebenen Art sind ferner bevorzugt (im wesentlichen) frei von Polyalkylenglykolen und / oder Mono-Alkylglycerinethern wie 1- oder 2-(C₁-C₂₄-Alkyl)glycerinether; oder von phosphorsauren Substanzen, insbesondere Phosphorsäurederivaten und / oder Polyalkylenglykolen und / oder Mono-Alkylglycerinethern wie 1- oder 2-(C₁-C₂₄-Alkyl)glycerinether.

Sie weisen einen pH - Wert zwischen 5 und 8,5 auf und sind daher auch anwenderfreundlich.

In einer weiteren besonders geeigneten Ausführungsform weist das alkoholhaltige Mittel keine sauren Phosphorsäurederivate und / oder Polyalkylenglykole oder Mono- Alkylglycerinether (1- oder 2-(C₁-C₂₄-Alkyl)glycerinether), und eine Einwirkzeit von weniger als 30 Sekunden auf unbelebten Oberflächen auf und enthält 35-70 Gew. %, vor allem 40-60 Gew. %, eines oder mehrerer Alkohole, 0,01 bis 10 Gew. %, bevorzugt 0,01 bis 5 Gew. %, eines oder mehrerer Zusätze, ausgewählt aus NH₃ (Ammoniak, d. h. wässrige Lösungen hiervon wie z.B. 25 %ig), einem oder mehreren amphoteren Tensiden, sowie als Rest Wasser. Insbesondere ist der Alkohol ausgewählt aus Ethanol, und / oder n-Propanol. Bevorzugter Zusatz sind insbesondere ein oder mehrere amphotere Tenside.

Alle Mengenangaben beziehen sich auf das Gewicht, sofern nicht anders angegeben.

Die vorliegende Erfindung betrifft insofern auch ein Verfahren zur Schnell- Desinfektion von unbelebten Oberflächen, das dadurch gekennzeichnet ist, dass man ein alkoholhaltiges Mittel, insbesondere der vorbeschriebenen und in den Ansprüchen genannten Art, auf die zu behandelnde unbelebte Oberfläche aufträgt und das Mittel über einen Zeitraum von weniger als 30 Sekunden einwirken lässt.

Die zu behandelnde Oberfläche kann ein ärztlicher oder zahnärztlicher Behandlungsstuhl, Operationstisch, Mobilar, Ausstattungsgegenstände oder ein sonstiges Medizinprodukt in Arzt- / Zahnarztpraxen sein. Dabei erfolgt eine Desinfektion (mikrobiologische Wirksamkeit) wie beschrieben gegenüber praxisrelevanten Keimen / Erregern, insbesondere gegen Bakterien wie z. B. Staphylococcus aureus, Pseudomonas aeruginosa und Enterococcus hirae, Pilzen, insbesondere Hefen wie Candida albicans, sowie behüllten / unbehüllten Viren wie z. B. HIV, HBV, HCV, HSV oder Rota- und Vaccinaviren. Die erfindungsgemäßen Mittel weisen insofern eine (mikrobiologische) Wirksamkeit gegenüber Bakterien, wie insbesondere grampositive oder gramnegative Bakterien wie z.B. Staphylococcus aureus, Pseudomonas aeruginosa und Enterococcus hirae, mit einer Keimreduktion von bevorzugt log RF ≥ 5, bzw. gegenüber Pilzen, wie insbesondere Hefen wie Candida albicans sowie behüllten und unbehüllten Viren bzw. behüllten und unbehüllten Viren wie z. B. HIV, HBV, HCV, Rota- oder Vaccinaviren oder auch Mykobakterien mit einer Keimreduktion bevorzugt von log RF ≥ 4 auf. (Keimreduktionswerte (log RF) gemäß

Standardmethoden der DGHM zur Prüfung chemischer Desinfektionsverfahren, qualitativer, insbesondere quantitativer Suspensionsversuch, 20 ° C, ohne Belastung, wie nachfolgend beschrieben).

Somit können die alkoholischen Mittel, insbesondere wie in den Ansprüchen beschrieben, verwendet werden zur Schnelldesinfektion von unbelebten Oberflächen, indem das auf die Oberfläche, vor allem solche wie oben genannt, aufgebrachte Mittel seine Wirksamkeit innerhalb eines Zeitraums von weniger als 30 Sekunden entfaltet, und innerhalb eines Zeitraums von weniger als 30 Sekunden wirksam ist gegen Bakterien wie z. B. Staphylococcus aureus, Pseudomonas aeruginosa und Enterococcus hirae, Pilze insbesondere Hefen wie Candida albicans, sowie behüllte/ unbehüllte Viren wie z. B. HIV, HBV, HCV, HSV oder auch gegen Rota- und Vaccinaviren oder auch Mykobakterien.

Bevorzugt weist das Mittel eine Wirksamkeit von 10 bis 25 Sekunden auf, bzw. wird über diesen Zeitraum verfahrensgemäß eingesetzt bzw. verwendet. Dazu kann es eingerieben oder aufgesprüht werden, wobei z. B. Sprühapplikatoren, getränkte oder imprägnierte textile Flächengebilde z. B. aus Cellulose, Baumwolle, synthetischen Polymeren ( z. B. PPVlies u.ä.) zum Einsatz kommen können.

### Herstellung

Die erfindungsgemäßen Mittel werden als gebrauchsfertige Lösung hergestellt, indem die beschriebenen Komponenten mit Wasser in bekannter Weise gemischt werden.

### Anwendung

Mit dem Schnelldesinfektionsmittel gemäß vorliegender Erfindung ist eine Desinfektion von unbelebten Oberflächen in kürzest nötiger Zeit möglich. Dazu kann das Mittel z. B. aus geeigneten Behältern wie Sprühflaschen ausgesprüht und / oder eingerieben werden (z. B. mit einem Tuch), oder nur eingerieben werden z. B. mit einem getränkten Tuch. Nach weniger als 30 Sekunden hat das Mittel seine Wirksamkeit entfaltet, Reste können auf übliche Weise entfernt werden. Das insbesondere Rückfetter- freie Mittel ist so konzipiert, dass bei der erfindungsgemäßen Einwirkzeit eine optimale gleichmäßige Verteilung auf der Oberfläche erfolgt und die gewünschte Desinfektion nicht durch Verdunstungseffekte beeinträchtigt wird. Dies war deshalb nicht zu erwarten, weil bisher eine Einwirkzeit von mindestens 30 Sekunden bis vorzugsweise 60 Sekunden, vor allem in Gegenwart bestimmter Zusätze, als erforderlich angesehen wurde. In den nachfolgend beschriebenen mikrobiologischen Tests ist jedoch aufgezeigt, dass eine kürzere Einwirkzeit ausreicht, ohne dass die Anwendungssicherheit gefährdet wird. Das Mittel ist insofern einfach, sicher und schnell in der Anwendung, da eine Beseitigung praxisrelevanter Keime wie grampositive oder gramnegative Bakterien z. B. Staphyloccoccus aureus, Pseudomonas aeruginosa und Enterococcus hirae als auch die Beseitigung von Mykobakterien, Pilzen, insbesondere Hefen wie Candida albicans, sowie von behüllten und unbehüllten Viren wie z. B. HBV, Vaccinaviren, Rotaviren und analogen Viren vorgenommen werden kann.

### Ausführungsformen und Anwendungsbeispiele

Die Erfindung wird anhand der nachfolgenden Beispiele näher erläutert.

### I. Herstellungsbeispiele

Die folgenden Mittel wurden wie oben beschrieben hergestellt.

| Beispiel 1 | |
|---|---|
| Ethanol | 46% |
| N-Alkylaminopropylglycin | 0,05% |
| Wasser | 53,95% |

| Beispiel 2 | |
|---|---|
| Ethanol | 46% |
| Didecyldimethylammoniumchlorid | 0,01 % |
| Wasser | 53,9% |

| Beispiel 3 | |
|---|---|
| Ethanol | 55% |
| N-Alkylaminopropylglycin | 0,03% |
| Wasser | 44,97% |

| Beispiel 4 | |
|---|---|
| Ethanol | 56% |
| Ammoniak, 25%ig | 0,05% |
| Wasser | 43,95% |

| Beispiel 5 | |
|---|---|
| Ethanol | 30% |
| n-Propanol | 15% |
| N-Alkylaminopropylglycin | 0,05% |
| Wasser | 54,95% |

| Beispiel 6 | |
|---|---|
| Ethanol | 30% |
| isopropanol | 15% |
| N-Alkylaminopropylglycin | 0,05% |
| Wasser | 54,95% |

| Beispiel 7 (Vergleich) | |
|---|---|
| n-Propanol | 46% |
| Natriumlaurylsulfat | 0,02% |
| Wasser | 53,98% |

| Beispiel 8 (Vergleich) | |
|---|---|
| Ethanol | 45% |
| Wasser | 54% |
| Isopropylmyristat | 1,0% |

### II. Wirkungsnachweise

Die biozide Wirkung wurde mit dem Testkeim a) Staphylococcus aureus und b) Pseudomonas aeruginosa im quantitativen Suspensionstest (gemäß den Standardmethoden der Deutschen Gesellschaft für Hygiene und Mikrobiologie (DGHM), bei 20 ° C, ohne Belastung, mit Mechanik ermittelt.

Die Standardmethoden der DGHM zur Prüfung chemischer Desinfektionsverfahren (Stand 1. September 2001) sind im mhp Verlag ISBN 3-88681-042-9 veröffentlicht. In ihnen werden die Methoden beschrieben, die zur Erstellung eines Desinfektionsmittelgutachtens gemäß den DGHM- Richtlinien zur Verfügung stehen. Sie definieren u.a. die Produktprüflösung, die Testorganismen, die Bestimmung der bakterostatischen und fungistatischen Wirkung sowie geeigneter Neutralisationsmittel, die Bestimmung der bakteriziden und fungiziden Wirkung im qualitativen bzw. quantitativen Suspensionsversuch und beschreiben die Methoden für die einzelnen Anwendungsbereiche z.B. Flächendesinfektion oder Chemische Instrumentendesinfektion.

Die Ergebnisse sind in nachfolgender Tabelle 1 zusammengefasst:

**Tabelle 1:**

| Beispiel Nr. | Einwirkzeit | Testkeim a) Log RF | Testkeim b) Log RF |
|---|---|---|---|
| 1 | 20 Sek. | >5,0 | > 5,0 |
| 2 | 25 Sek. | >5,0 | > 5,0 |
| 3 | 15 Sek. | > 5,0 | > 5,0 |
| 4 | 25 Sek. | > 5,0 | > 5,0, |
| 5 | 20 Sek.. | >5,0 | > 5,0 |
| 6 | 20 Sek. | >5,0 | > 5,0 |
| 7* | 30 Sek. | 4,8 | 4,2 |
| 8* | 30 Sek. | 4,7 | 4,1 1 |

| | | | |
|---|---|---|---|
| * Vergleichsbeispiele | | | |

## Patentansprüche

1. Alkoholhaltiges Mittel zur Schnell- Desinfektion unbelebter Oberflächen, **dadurch gekennzeichnet, dass** es eine mikrobiologische Wirksamkeit innerhalb eines Zeitraums von weniger als 30 Sekunden aufweist.

2. Mittel gemäß Anspruch 1, **dadurch gekennzeichnet, dass** es ein oder mehrere Alkohole, ausgewählt aus Ethanol, n- Propanol, Isopropanol oder Mischungen hiervon aufweist.

3. Mittel gemäß einem der Ansprüche 1 oder 2, **dadurch gekennzeichnet, dass** es 0,01 bis 10 Gew.% eines oder mehrerer Zusätze, ausgewählt aus NH₃, (Ammoniak), einem oder mehreren amphoteren Tensiden, einem oder mehreren nicht ionischen Tensiden, einem oder mehreren pH- Wert Regulatoren, einem oder mehreren bioziden Wirkstoffen, Parfüm-Farbstoffen, sowie als Rest Wasser aufweist.

4. Mittel gemäß Anspruch 3, **dadurch gekennzeichnet, dass** es 35-70 Gew.%, eines oder mehrerer Alkohole, 0,01 bis 10 Gew. % eines oder mehrerer Zusätze, ausgewählt aus NH₃ (Ammoniak), einem oder mehreren amphoteren Tensiden, einem oder mehreren pH- Wert Regulatoren, sowie als Rest Wasser aufweist.

5. Mittel gemäß Anspruch 4, **dadurch gekennzeichnet, dass** es 40-60 Gew. % eines oder mehrerer Alkohole, 0,01 bis 5 Gew. % eines oder mehrerer Zusätze, ausgewählt aus einem oder mehreren amphoteren Tensiden, sowie als Rest Wasser aufweist.

6. Mittel gemäß einem der Ansprüche 1 bis 5, **dadurch gekennzeichnet, dass** es eine mikrobiologische Wirksamkeit gegen grampositive oder gramnegative Bakterien wie z.B. Staphylococcus aureus, Pseudomonas aeruginosa und Enterococcus hirae, mit einer Keimreduktion von log RF ≥ 5, bzw. gegenüber Pilzen, wie insbesondere Hefen wie Candida albicans bzw. gegen behüllte und unbehüllte Viren wie z. B. HIV, HBV, HCV, Rota- oder Vaccinaviren oder auch Mykobakterien mit einer Keimreduktion log RF ≥ 4 aufweist.

7. Mittel gemäß einem der Ansprüche 1 bis 6, **dadurch gekennzeichnet, dass** es im wesentlichen frei ist von Polyalkylenglykolen.

8. Mittel gemäß einem der Ansprüche 1 bis 7, **dadurch gekennzeichnet, dass** es im wesentlichen frei ist von Mono- Alkylglycerinethern.

9. Mittel gemäß einem der Ansprüche 1 bis 8, **dadurch gekennzeichnet, dass** es 38 bis 65 Gew. % Alkohol, 0,01 bis 5 Gew. Zusätze sowie Wasser aufweist.

10. Mittel gemäß einem der Ansprüche 1 bis 9, **dadurch gekennzeichnet, dass** es im wesentlichen frei ist von Polyalkylenglykolen und/ oder sauren Phosphorsäurederivaten und Mono- Alkylglycerinethern.

11. Verfahren zur Schnell- Desinfektion von unbelebten Oberflächen, **dadurch gekennzeichnet, dass** man ein alkoholhaltiges Mittel auf die zu behandelnde Oberfläche mechanisch aufträgt und das Mittel über einen Zeitraum von weniger als 30 Sekunden einwirken lässt.

12. Verfahren gemäß Anspruch 11, **dadurch gekennzeichnet, dass** man ein Mittel gemäß einem der Ansprüche 1 bis 10 auf die zu behandelnde Oberfläche aufträgt und das Mittel über einen Zeitraum von weniger als 30 Sekunden einwirken lässt.

13. Verfahren gemäß Anspruch 11 oder 12, **dadurch gekennzeichnet, dass** die zu behandelnde Oberfläche ein ärztlicher oder zahnärztlicher Behandlungsstuhl, Operationstisch, Mobilar, Ausstattungsgegenstände in Arzt-/Zahnarztpraxen oder ein sonstiges Medizinprodukt ist.

14. Verfahren gemäß einem der Ansprüche 11 bis 13, **dadurch gekennzeichnet, dass** das Auftragen auf die Oberfläche durch Einreiben, Sprühen oder eine Kombination hiervon erfolgt.

15. Verwendung eines alkoholischen Mittels zur Schnell- Desinfektion von unbelebten Oberflächen, **dadurch gekennzeichnet, dass** das auf die Oberfläche aufgebrachte Mittel seine Wirksamkeit innerhalb eines Zeitraums von weniger als 30 Sekunden entfaltet.

16. Verwendung gemäß Anspruch 15, **dadurch gekennzeichnet, dass** man ein Mittel gemäß einem der Ansprüche 1-10 einsetzt.

17. Verwendung gemäß einem der Ansprüche 15 oder 16, **dadurch gekennzeichnet, dass** man das Mittel 10 bis 25 Sekunden einwirken lässt.

18. Verwendung gemäß einem der Ansprüche 15 bis 17 durch Aufsprühen, Einreiben oder eine Kombination hiervon.

19. Verwendung gemäß einem der Ansprüche 15 bis 18, **dadurch gekennzeichnet, dass** die zu behandelnde Oberfläche ein ärztlicher oder zahnärztlicher Behandlungsstuhl, Operationstisch, Mobilar, Ausstattungsgegenstände in Arzt-/Zahnarztpraxen oder ein sonstiges Medizinprodukt ist.
